# EUROPÄISCHE PATENTSCHRIFT

(11) **EP 0 542 680 B1**
(45) Veröffentlichungstag und Bekanntmachung des Hinweises auf die Patenterteilung: **28.01.1998**
(21) Anmeldenummer: 92810846.3
(22) Anmeldetag: 03.11.1992
(51) Int. Cl.: C07F 7/08, C07F 7/18, C07D 319/08, C07D 321/00, C07D 327/04, C07C 43/178, C07D 473/00

(54) **Geschützte Formylpentandiole, Hydroxymethylpentandiole, Verfahren zur deren Herstellung und Zwischenprodukte**
Protected formylpentanediols and protected hydroxymethylpentanediols, process for their preparation and intermediates
Formylpentanedioles protégés et hydroxyméthylpentanedioles protégés, procédé pour leur préparation et intermédiaires

(30) Priorität: 12.11.1991 CH 3291/91
(43) Veröffentlichungstag der Anmeldung: 19.05.1993
(73) Patentinhaber: Novartis AG, 4058 Basel (CH)
(72) Erfinder: Moser, Heinz, Dr., CH-4313 Möhlin (CH)

(56) Entgegenhaltungen:
- EP-A- 0 249 859
- JOURNAL OF ORGANIC CHEMISTRY, Band 54, 1989; Y. INOUE et al., Seiten 5268-5272
- TETRAHEDRON LETTERS, Band 7, Nr. 6, 1986; P. RAVENSCROFT et al., Seiten 747-748
- SYNTHESIS, Nr. 9, 1985; M. LALONDE et al., Seiten 817-845

## Beschreibung

Die Erfindung betrifft geschützte 4-Formyl-1,3-pentandiole, geschützte 4-Hydroxymethyl-1,3-pentandiole, ein Verfahren zu deren Herstellung und beim Herstellungsverfahren verwendete geschützte Pent-4-en-1,3-diole als Zwischenprodukte.

Carbacyclische Nukleosidanaloge haben auf Grund Ihrer antiviralen Eigenschaften und als Bausteine für Oligonukleotide mit Antisense-Eigenschaften Interesse erlangt, siehe zum Beispiel M. Bodenteich et al., Nucleosides & Nucleotides, 6(182), Seiten 233-237 1987, A. Szemzo et al., Tetrahedron Letters, Vol. 31, No. 10, Seiten 1463-1466 (1990), K. Biggadike et al., J. Chem. Soc., Chem. Commun., Seiten 1083-1084 (1987), P. Ravenscroft et al., Tetrahedron Letters, Vol. 27, No. 6, Seiten 747-748 (1986), J. Balzarini et al., J. Med. Chem., Vol. 32, Seiten 1861-1865 (1989), J. Beres et al., J. Med. Chem., Vol. 33, Seiten 1353-1360 (1990) und V. E. Marquez et al., Med. Res. Rev., Vol. 6, Seiten 1-40 (1986). Die bekannten Synthesemethoden sind sehr aufwendige Mehrstufenverfahren, mit denen die gewünschten Nukleosidanalogen in nur geringen Ausbeuten erhalten werden. Für die Synthese der carbacyclischen, sowohl racemischer als auch enantiomerenreiner Nukleosidanalogen in hohen Ausbeuten ist noch kein befriedigendes Verfahren gefunden worden. Ein Verfahren zur gleichzeitigen Herstellung von solchen Nukleosidanalogen der natürlichen und unnatürlichen Reihe ist ebenfalls nicht bekannt.

Es wurde nun gefunden, dass man solche Nukleosidanaloge in hohen Ausbeuten und Reinheiten sowohl in racemischer als auch enantioselektiver Form herstellen kann, wenn man geschützte 4-Formyl-1,3-pentandiole als Ausgangsverbindungen auswählt, wobei man gleichzeitig Nukleosidanaloge der natürlichen und unnatürlichen Reihe herstellen kann.

Ein Gegenstand vorliegender Erfindung sind Verbindungen der Formeln Ia und Ib 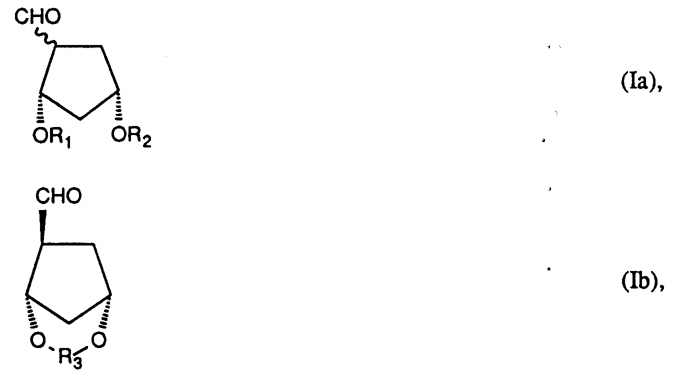 worin R₁ und R₂ unabhängig voneinander lineares oder verzweigtes C₁-C₁₂-Alkyl, Cycloalkyl mit 5 bis 8 Ringkohlenstoffatomen, Phenylalkyl mit 1 bis 4 C-Atomen in der Alkylengruppe bedeuten, wobei die cyclischen Reste unsubstituiert oder mit C₁-C₄-Alkyl oder C₁-C₄-Alkoxy substituiert sind, oder R₁ und R₂ unabhängig voneinander -SiR₄R₅R₆ darstellen, worin R₄, R₅ und R₆ unabhängig voneinander C₁-C₁₂-Alkyl, Phenyl, Benzyl oder mit C₁-C₄-Alkyl oder C₁-C₄-Alkoxy substituiertes Phenyl oder Benzyl darstellen, und R₃ eine Brückengruppe bedeutet, die zusammen mit dem Rest -OCH-CH₂-CHO-, an den sie gebunden ist, einen 6- bis 8-gliedrigen Ring bildet.

R₁ und R₂ stellen bevorzugt gleiche Reste dar. R₁ und R₂ stellen als Alkyl bevorzugt lineares oder verzweigtes C₁-C₈-Alkyl, besonders bevorzugt C₁-C₄-Alkyl dar. Beispiele für Alkyl sind Methyl, Ethyl, und die Isomeren von Propyl, Butyl, Pentyl, Hexyl, Heptyl, Octyl, Nonyl, Decyl, Undecyl und Dodecyl. Bevorzugte Beispiele sind Methyl, Ethyl, n- und i-Propyl, n-, i- und t-Butyl.

R₁ und R₂ enthalten als Cycloalkyl bevorzugt 5 oder 6 Ringkohlenstoffatome. Einige Beispiele für R₁ und R₂ als gegebenenfalls substituiertes Cycloalkyl sind Cyclopentyl, Cyclohexyl, Cyclooctyl, Methylcyclopentyl, Methylcyclohexyl und Methoxycyclohexyl.

R₁ und R₂ stellen als Phenylalkyl bevorzugt gegebenenfalls substituiertes Phenylethyl, besonders bevorzugt Benzyl dar. Einige bevorzugte Beispiele sind Benzyl, Methylbenzyl, Dimethylbenzyl und Alkoxybenzyl.

In einer bevorzugten Ausführungsform stellen R₁ und R₂ gleiche Reste -SiR₄R₅R₆ dar. R₄, R₅ und R₆ bedeuten bevorzugt unabhängig voneinander C₁-C₁₂-Alkyl oder Phenyl. Besonders bevorzugt sind R₄, R₅ und R₆ C₁-C₆-Alkyl. Beispiele für Alkyl sind zuvor erwähnt worden. Bevorzugte Beispiele für Reste -SiR₄R₅R₆ sind Trimethylsilyl, Triethylsilyl, Tri-n-propylsilyl, Tri-i-propylsilyl, Tri-n-butylsilyl, Triphenylsilyl, t-Butyldiphenylsilyl, Phenyldimethylsilyl, i-Propyldimethylsilyl, t- oder n-Butyldimethylsilyl, n-Hexyldimethylsilyl, n-Octyldimethylsilyl, n-Dodecyldimethylsilyl, und (1,1,2,2-Tetramethylethyl)dimethylsilyl.

Geeignete Brückengruppen für R₃ sind zum Beispiel Alkyliden mit 1 bis 20 C-Atomen, Cycloalkyliden mit 5 bis 8 Ringkohlenstoffatomen, und Reste der Formeln R₇R₈Si=und -SiR₇R₈-O-R₇R₈Si-, worin R₇ und R₈ unabhängig voneinander lineares oder verzweigtes C₁-C₁₂-Alkyl, Phenyl, Benzyl oder mit C₁-C₄-Alkyl oder C₁-C₄-Alkoxy substituiertes Phenyl oder Benzyl darstellen.

R₃ enthält als Alkyliden bevorzugt 1 bis 14 C-Atome. Beispiele für Alkyliden sind Methylen, Phenylmethylen, Diphenylmethylen, Phenyl-methyl-methylen, Ethyliden, 1,1-oder 2,2-Propyliden, 1,1- oder 2,2-Butyliden, 1,1-, 2,2- oder 3,3-Pentyliden, 3,3-Dimethyl-2,2-butyliden, 1,1-, 2,2- und 3,3-Hexyliden, Octyliden und Dodecyliden. Besonders bevorzugt handelt es sich bei R₃ als Alkyliden um C₁-C₆-Alkyliden und Benzyliden. Bei R₃ als Cycloalkyliden handelt es sich bevorzugt um Cyclopentyliden und Cyclohexyliden.

Im Rest R₇R₈Si= und im Rest -SiR₇R₈-O-R₇R₈Si- bedeuten R₇ und R₈ bevorzugt unabhängig voneinander C₁-C₁₂-Alkyl oder Phenyl und insbesondere bevorzugt C₁-C₆-Alkyl oder Phenyl. Besonders bevorzugt stellen R₇ und R₈ gleiche Reste dar. Beispiele für Alkyl sind zuvor erwähnt worden. Bevorzugte Beispiele für diese Reste sind Dimethylsilyliden, Diethylsilyliden, Di-n-propylsilyliden, Di-i-Propylsilyliden, Di-n-butylsilyliden, Di-i-butylsilyliden, Di-t-butylsilyliden, Diphenylsilyliden, Phenylmethylsilyliden, i-Propylmethylsilyliden, t- oder n-Butylmethylsilyliden, n-Hexylmethylsilyliden, n-Octylmethylsilyliden, n-Dodecylmethylsilyliden und (1,1,2,2-Tetramethylethyl)methylsilyliden, und Tetramethyl-,Tetraethyl, Tetra-n- oder -i-propyl-, Tetra-n- oder Tetra-i- oder Tetra-t-Butyl-, 1,3-Dimethyl-1,3-di-t-butyl-2-oxa-1,3-disila-prop-1,3-diyl.

Eine besonders bevorzugte Ausführungsform sind die Verbindungen der Formel Ib, insbesondere solche, worin R₃ einen Rest R₇R₈Si= oder einen Rest -SiR₇R₈-O-R₇R₈Si- bedeutet, worin R₇ und R₈ bevorzugt gleiche Reste darstellen und für C₁-C₆-Alkyl stehen.

Ein weiterer Gegenstand der Erfindung ist ein Verfahren zur Herstellung der Verbindungen der Formeln Ia und Ib, das dadurch gekennzeichnet ist, dass man
a) in einer ersten Stufe 1 Äquivalent Cis-1,3-Pent-4-endiol der Formel 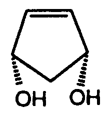
a1) mit 2 Äquivalenten R₁X, R₂X oder Mischungen hiervon umsetzt, oder
a2) mit einem Äquivalent XR₃X umsetzt, worin R₁, R₂ und R₃ die zuvor angegebene Bedeutung haben und X oder zwei X gemeinsam für eine Abgangsgruppe steht, und
b) in einer zweiten Stufe die erhaltenen Verbindungen der Formeln IIa und IIb 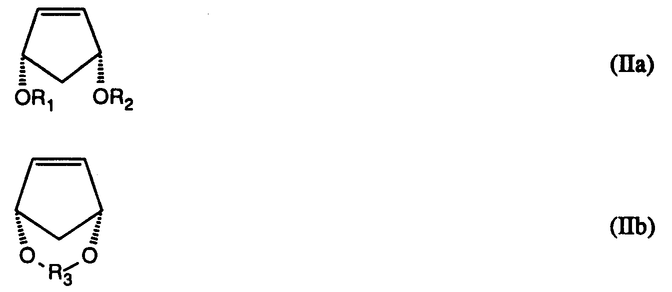
in Gegenwart eines Katalysators bei erhöhter Temperatur und unter Druck mit einem Gemisch aus H₂ und CO zu Verbindungen der Formeln Ia und Ib hydroformyliert.

Die Verbindungen der Formel IIb sind neu und stellen einen weiteren Gegenstand der Erfindung dar. Für die Brückengruppe R₃ gelten die zuvor aufgeführten Bevorzugungen und Aufzählungen. In den Verbindungen der Formel IIa stellen R₁ und R₂ bevorzugt gleiche Gruppen -SiR₄R₅R₆ dar.

Das verwendete 1,3- Pent-4-endiol ist bekannt und seine Herstellung ist von C. Kaneko in Synthesis, Seite 876 (1974) beschrieben.

Geeignete Abgangsgruppen X sind zum Beispiel Halogenid, besonders Cl^{⊖}, Br^{⊖}, sowie CF₃COO^{⊖}, RSO₃^{⊖}, SO₄^{2⊖}, PO₄^{3⊖}, CH₃CO₂^{⊖}, OH^{⊖}, worin R für C₁-C₆-Alkyl oder teilweise oder vollständig mit Fluor substituiertes C₁-C₆-Alkyl, oder unsubstituiertes oder mit C₁-C₄-Alkyl, C₁-C₄-Alkoxy oder Halogen (zum Beispiel F, Cl oder Br) substituiertes Phenyl oder Benzyl bedeutet. Bei den in der Verfahrensstufe a2) erwähnten Verbindungen, worin zwei X eine Abgangsgruppe darstellen, handelt es sich insbesondere um Aldehyde und Ketone, die zur Acetalisierung beziehungsweise Ketalisierung der verwendeten 1,3-Pentendiole eingesetzt werden. Die Verbindungen R₁X, R₂X und XR₃X sind allgemein bekannt und in der Literatur beschrieben. Bevorzugte Abgangsgruppen in Silylierungsreagenzien sind Cl^{⊖}, Br^{⊖}, CF₃SO₃^{⊖} und CF₃COO^{⊖}.

Die Reaktionen in den Verfahrensstufen a) und b) können zweckmässig in Gegenwart eines inerten Lösungsmittels durchgeführt werden.

Geeignete inerte Lösungsmittel sind zum Beispiel polare oder unpolare und bevorzugt aprotische Lösungsmittel, die alleine oder in Mischungen aus mindestens zwei Lösungsmittel verwendet werden können. Beispiele sind: Ether (Dibutylether, Tetrahydrofuran, Dioxan, Ethylenglykoldiethyl- oder -dimetylether, Diethylenglykoldimethylether, Diethylenglykoldiethylether, Triethylenglykoldimethyletehr), halogenierte Kohlenwasserstoffe (Methylenchlorid, Chloroform, 1,2-Dichlorethan, 1,1,1-Trichlorethan, 1,1,2,2-Tetraclorethan), Carbonsäureester und Lactone (Essigsäureethylester, Propionsäuremethylester, Benzoesäureethylester, 2-Methoxyethylacetat, γ-Butyrolacton, δ-Valerolacton, Pivalolacton), Carbonsäureamide und Lactame (N,N-Dimethylformamid, N,N-Diethylformamid, N,N-Dimethylacetamid, Tetramethylharnstoff, Hexamethylphosphorsäuretriamid, γ-Butyrolactam, ε-Caprolactam, N-Methylpyrrolidon, N-Acetylpyrrolidon, N-Methylcaprolactam), Sulfoxide (Dimethylsulfoxid), Sulfone (Dimethylsulfon, Diethylsulfon, Trimethylensulfon, Tetramethylensulfon), tertiäre Amine (Triethylamin, N-Methylpiperidin, N-Methylmorpholin), aromatische Kohlenwasserstoffe wie zum Beispiel Benzol oder substituierte Benzole (Chlorbenzol, o-Dichlorbenzol, 1,2,4-Trichlorbenzol, Nitrobenzol, Toluol, Xylol) und Nitrile (Acetonitril, Propionitril, Benzonitril, Phenylacetonitril), sowie aliphatische oder cycloaliphatische Kohlenwasserstoffe (Pentan, Petrolether, Hexan, Cyclohexan und Methylclohexan).

Bevorzugte Lösungsmittel sind halogenierte aliphatische Kohlenwasserstoffe, aromatische Kohlenwasserstoffe, Ether und Nitrile, zum Beispiel Methylenchlorid, Chloroform, Benzol, Toluol, Acetonitril, Diethylether, Dibutylether, Tetrahydrofuran und Dioxan.

Stellen die Verbindungen R₁X und R₂X Alkohole dar oder stellt XR₃X einen Aldehyd oder ein Keton dar, so werden vorteilhaft in an sich bekannter Weise Metallsalz-oder Metallkomplexsalz-Katalysatoren mitverwendet, zum Beispiel von Pd, Pt, Rh und Ru. Beispiele sind Tris(triphenylphosphino)rhodiumchlorid oder Pt(H₂O)₂Cl₂ oder [(1, 1,1-Tris(diphenylphosphinomethyl)ethan)Ru(CH₃CN)₃]^{2⊕} CF₃SO₃^{⊖})₂. Ferner kann es zweckmässig sein, bei der Verwendung von Alkoholen, Aldehyden und Ketonen das entstehende Reaktionswasser zu entfernen, zum Beispiel durch azeotrope Destillation oder die Zugabe wasserbindender Mittel.

Die Reaktionen der Stufe a) können bei Temperaturen von -80 °C bis zu 250 °C durchgeführt werden, vorzugsweise von -20 °C bis zur Rückflusstemperatur des verwendeten Lösungsmittels. Die Wahl der Reaktionstemperatur hängt im wesentlichen von der Reaktivität der Verbindungen R₁X, R₂X und XR₃X ab.

Die Verbindungen der Formeln IIa und IIb können nach üblichen Methoden isoliert und gereinigt werden.

Die Hydroformylierung der Reaktionsstufe b) wird vorteilhaft bei einer Temperatur von 40 bis etwa 200 °C und bei einem Druck von 1 bis 20, bevorzugt 2 bis 10 MPa durchgeführt. Geeignete Lösungsmittel sind zuvor erwähnt worden. Bevorzugte Lösungsmittel sind Ether wie zum Beispiel Tetrahydrofuran. Als Katalysatoren sind zum Beispiel gegebenenfalls komplexierte Edelmetallsalze geeignet, wie zum Beispiel Tris(triphenylphosphino)rhodiumchlorid. Die Hydroformylierung von ethylenisch ungesättigten organischen Verbindungen ist allgemein bekannt. Das Mischungsverhältniss von H₂ zu CO beträgt bevorzugt 1:1.

Die Verbindungen der Formel Ia werden als Gemisch von Cis- und Transverbindungen erhalten, das zur Gewinnung der Transverbindung zum Beispiel durch chromatographische Methoden getrennt werden kann. Bei Verwendung der Verbindungen der Formel IIb verläuft die Hydroformylierung überraschend regioselektiv, so dass man nur die gewünschten Transverbindungen direkt in hohen Ausbeuten und Reinheiten erhält. Die Verwendung der Verbindungen der Formel IIb ist daher bevorzugt. Die neuen Verbindungen der Formeln Ia und Ib werden als Racemat erhalten, das nach üblichen Methoden getrennt werden kann. Gegenstand der Erfindung sind auch die beiden Enantiomeren.

Die Verbindungen der Formeln Ia und Ib eignen sich hervorragend als Ausgangsprodukte zur Herstellung von carbacyclischen Nukleosiden der natürlichen und unnatürlichen Reihe, beziehungsweise deren enantiomeren Gemischen. Die Verbindungen der Formeln Ia und Ib müssen hierzu nicht isoliert werden, sondern können direkt weiterverarbeitet werden. Es ist daher bevorzugt, die Hydroformylierung in einem Ether als Lösungsmittel durchzuführen, da die nachfolgende Hydrierung der Formylgruppe zweckmässig im gleichen Lösungsmittel durchgeführt wird.

Ein weiterer Gegenstand der Erfindung sind Verbindungen der Formeln IIIa und IIIb 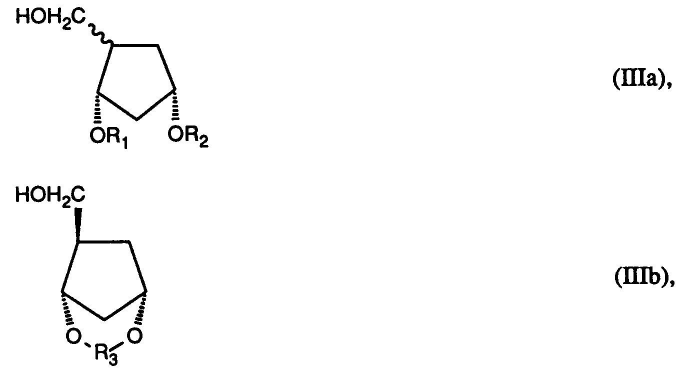 worin R₁, R₂ und R₃ die zuvor angegebenen Bedeutungen haben, in Form Ihrer Racemate und Enantiomeren, sowie deren Hydroxylgeschützte geschützte Derivate.

Die Verbindungen sind durch die Hydrierung der Verbindungen der Formeln Ia und Ib erhältlich. Die Hydrierung kann in an sich bekannter Weise katalytisch oder mit Metallhydriden wie zum Beispiel LiH, CaH₂, NaBH₄ oder LiAlH₄ erfolgen.

Geschützt bedeutet, dass die Hydroxylgruppe mit einer abspaltbaren Schutzgruppe derivatisiert ist. Solche Schutzgruppen und Verfahren zur Derivatisierung sind in der Zuckerchemie allgemein bekannt. Beispiele für solche Schutzgruppen sind: lineares oder verzweigtes C₁-C₈-, besonders C₁-C₄-Alkyl, zum Beispiel Methyl, Ethyl, n- und i-Propyl, n-, i- und t-Butyl; C₇-C₁₂-Aralkyl, zum Beispiel Benzyl, Methylbenzyl, Methoxybenzyl, Brombenzyl; Diphenylmethyl, Trityl, 4-'Monomethoxytrityl, 4',4''-Dimethoxytrityl, Pixyl; Trialkylsilyl mit 3 bis 20, besonders 3 bis 12 C-Atomen, zum Beispiel Trimethylsilyl, Triethylsilyl, Tri-n-propylsilyl, i-Propyl-dimethylsilyl, t-Butyl-dimethylsilyl, t-Butyl-diphenylsilyl, n-Octyl-dimethylsilyl, (1,1,2,2-Tetramethylethyl)-dimethylsilyl; C₂-C₁₂-, besonders C₂-C₈-Acyl, wie zum Beispiel Acetyl, Propanoyl, Butanoyl, Pentanoyl, Hexanoyl und Benzoyl; R₁₀-SO₂-, worin R₁₀ C₁-C₁₂-Alkyl, besonders C₁-C₆-Alkyl, C₅- oder C₆-Cycloalkyl, Phenyl, Benzyl, C₁-C₁₂- und besonders C₁-C₄-Alkylphenyl, oder C₁-C₁₂- und besonders C₁-C₄-Alkylbenzyl, oder Halogenphenyl oder Halogenbenzyl bedeutet, zum Beispiel Methyl-, Ethyl-, Propyl-, Butyl-, Phenyl-, Benzyl-, p-Brom-, p-Methoxy- und p-Methylphenylsulfonyl; C₁-C₁₂-, bevorzugt C₁-C₈-Alkoxycarbonyl, zum Beispiel Methoxy-, Ethoxy-, n- oder i-Propoxy- oder n-, i- oder t-Butoxycarbonyl, Phenyloxycarbonyl, Benzyloxycarbonyl, Methyl- oder Methoxy- oder Chlorphenyloxycarbonyl oder -benzyloxycarbonyl.

Für R₁, R₂ und R₃ gelten ebenfalls die zuvor erwähnten Bevorzugungen und Aufzählungen.

Die Cis- und Transisomeren der Formel IIIa und die Racemate können nach an sich bekannten chromatographischen Methoden getrennt werden. Die Verbindungen der Formel IIIb sind bevorzugt.

Die weitere Umsetzung der Verbindungen der Formel IIIa und IIIb zu carbacyclischen Nukleosiden kann zum Beispiel auf nachfolgende Weise erfolgen:

Die Hydroxylgruppe der Verbindungen der Formel IIIa und IIIb wird in an sich bekannter Weise geschützt, zum Beispiel mit aus der Zuckerchemie bekannten Schutzgruppen. Geeignet und als Beispiel erwähnt sei Trityl. Aus den geschützten Verbindungen können die Gruppen R₁ und R₂ beziehungsweise R₃ in bekannter Weise abgespalten werden, zum Beispiel mit wässrigen Säuren oder Basen oder bei Si-haltigen Resten mit Tetrabutylammoniumfluorid. Das erhaltene geschützte Diol der Formel IV 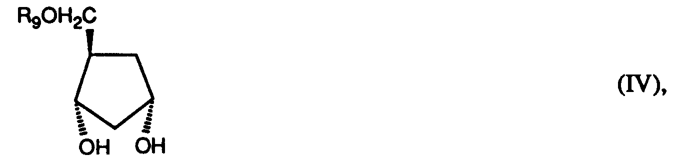 worin R₉ eine Schutzgruppe bedeutet, kann als Racemat weiterverwendet werden oder mittels einer enzymatischen katalysierten Monoacylierung mit Vinylacetat in Gegenwart der Lipase von Pseudomonas Fluorescens oder Chromobakterium viscosum in hohen Ausbeuten in ein Isomerengemisch mit den Regioisomeren unterschiedlicher absoluter Konfiguration der Formeln Va und Vb übergeführt werden: 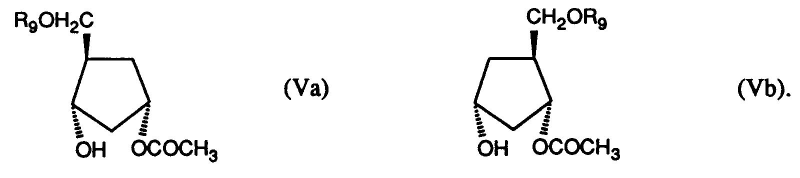 R₉ stellt bei dieser enzymatischen Reaktion bevorzugt eine räumlich anspruchsvolle Schutzgruppe dar, zum Beispiel unsubstituiertes oder mit C₁-C₄-Alkyl oder C₁-C₄-Alkoxy substituiertes Diphenylmethyl und besonders Triphenylmethyl (Trityl), sowie t-Butyldiphenylsilyl.

Das erhaltene Isomerengemisch kann in einfacher Weise durch Chromatographie an Kieselgel vollständig getrennt werden. Die so in praktisch quantitativen Ausbeuten erhaltenen Regioisomeren werden in hoher Enantiomerenreinheit (ee mindestens 98 %) erhalten. Die Umsetzung der Verbindungen der Formeln Va und Vb mit Basen wie zum Beispiel Ammoniak, Aminen oder Alkalimetallbasen in alkoholischer Lösung führt in praktisch quantitativen Ausbeuten zu den enantiomeren Diolen der Formeln IVa und IVb, 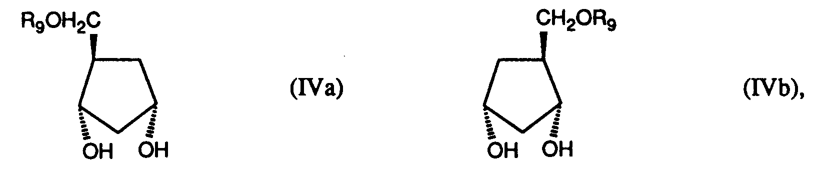 wobei es sich bei den Verbindungen der Formel IVa um die (+)-Enantiomeren der natürlichen Reihe und entsprechend bei den Verbindungen der Formel IVb um die (-)-Enantiomeren der unnatürlichen Reihe handelt.

Die Racemate der Formel IV und die Enantiomeren der Formeln IVa und IVb können mit SOCl₂ in Gegenwart von tertiären Aminen zu Sulfiten umgesetzt werden, die man zu den entsprechenden Sulfaten der Formeln VI in Form Ihrer Racemate oder Enantiomeren oxidieren kann, zum Beispiel nach der von B. M. Kim und K. B. Sharpless in Tetrahedron Letters 30, Seite 655 (1989) beschriebenen Methode:

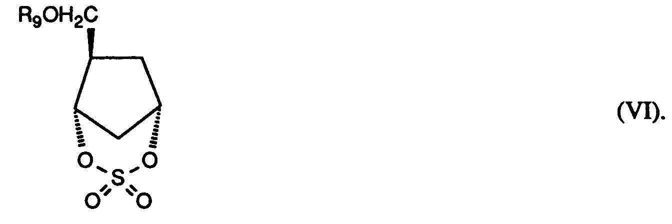

Die Verbindungen der Formel VI können direkt mit Basen wie zum Beispiel Adenin oder Thymin umgesetzt werden. Die Umsetzung ist regiospezifisch und im Falle des Adenins oder Thymins werden nach der hydrolytischen Abspaltung der Sulfatgruppe und der Abspaltung der Schutzgruppe nach bekannten Methoden Nukleoside der Formel 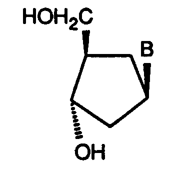 gebildet, worin B für 9-Adenyl oder 1-Thymidyl steht. Diese pharmazeutischen Wirkstoffe und ihre Wirksamkeit als antivirale Wirkstoffe sind von V. E. Marquezet al. in Med. Res. Rev., Vol. 6, Seiten 1-40 (1986). Die Nukleoside können auch zur Herstellung von Oligonukleotiden verwendet werden, die bekannterweise ebenfalls biologische Aktivitäten aufweisen, vergleiche E. Uhlmann et al. in Chem. Rev., Vol. 90, No.4, Seiten 543-584 (1990).

Die carbacyclischen Nukleoside werden trotz der Mehrstufensynthese in hohen Gesamtausbeuten erhalten. Der besondere Vorteil der Synthesemethode ist die Möglichkeit, gleichzeitig Racemate von Nukleosiden und deren Enantiomeren sowohl der unnatürlichen als auch natürlichen Reihe herzustellen. Das Verfahren ist für den industriellen Masstab geeignet.

Die nachfolgenden Beispiele erläutern die Erfindung näher.

### A) Herstellungsbeispiele

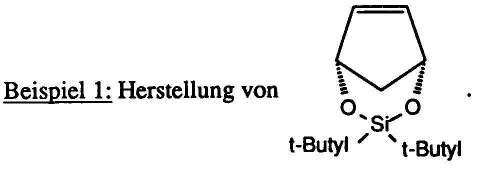

0,91 g (9,1 mMol) cis-4-Cyclopenten- 1,3-diol und 20 ml wasserfreies Methylenchlorid werden unter Argonatmosphäre in einem Reaktionskolben vorgelegt und auf 0 °C gekühlt. Unter Rühren werden 3,17 ml (27,3 mMol) Lutidin zugegeben und danach innerhalb von 10 Minuten 4,42 g (10 mMol) Di-t-butylsilylditriflat. Das Reaktionsgemisch wird 10 Minuten bei 0 °C gerührt, dann das Eisbad entfernt und 30 Minuten lang weitergerührt. Danach entfernt man das Lösungsmittel unter Vakuum und versetzt den öligen Rückstand unter kräftigem Rühren mit 20 ml Hexan. Das gebildete kristalline Lutidiniumaiflat wird abfiltriert das Lösungsmittel aus dem Filtrat verdampft. Man chromatographiert den Rückstand an Silikagel mit Hexan/Diethylether (20:1) und erhält nach Abdampfen des Lösungsmittels und Trocknen des Rückstands (Raumtemperatur, 30 Minuten, 1,3 Pa) 1,4 g (65 %) eines farblosen Öls. ¹H-NMR (250 MHz, CDCl₃): 0,96 und 1,04 (2 s, 2 (CH₃CSi); 1,81 [dt, J=12,0 und 3,0, H-C(2)]; 2,49 [d, J=12,0, HC(2)].
Eine Wiederholung des Verfahrens mit der fünffachen Menge ergibt eine Ausbeute von 80 % der Theorie.

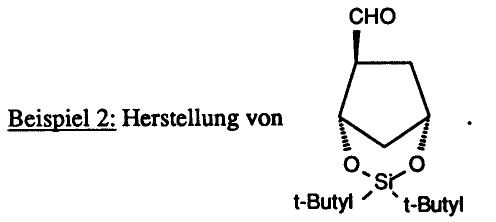

1 g (4,16 mMol) der Verbindung gemäss Beispiel 1, 15,4 mg (0,4 Mol-%) RhCl[P(C₆H₅)₃]₃ und 30 ml Tetrahydrofuran werden in einem 50 ml goldbeschichteten Autoklaven vorgelegt und dann unter einer H₂/CO-Atmosphäre bei 8 MPa und 80 °C 5 Stunden lang erhitzt. Nach dem Abkühlen wird das Lösungsmittel im Vakuum abgedampft und der Rückstand an Silikagel chromatographiert (Hexan/Essigsäureethylester 9:1). Danach entfernt man die Lösungsmittel im Vakuum und trocknet den Rückstand eine Stunde bei Raümtemperatur und 8 Pa. Man erhält 1,07 g (95 %) der Titelverbindung als farbloses Öl. ¹H-NMR (250MHz, CDCl₃):1,05 und 1,06 (2s, 2 (CH₃CSi); 1,24 [ddd, J=14,0, 3,0 und 3,0, H-C(2)]; 2,44 [d, J=14,0, HC(2)]; 9,79 (s, HCO).

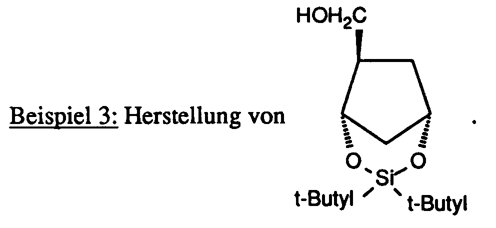

491 mg (1,82 mMol) der Verbindung gemäss Beispiel 2 werden in 10 ml Tetrahydrofuran/H₂O (9:1) gelöst und auf 0 °C gekühlt. Unter Rühren werden dann 34,5 mg (0,91 mMol) NaBH₄ auf einmal zugegeben und noch 10 Minuten weitergerührt. Danach wird das Lösungsmittel verdampft, der Rückstand zweimal mit je 30 ml Essigsäweethylester extrahiert, und die organische Phase mit je 20 ml 2,5-prozentiger wässriger NaHCO₃ und konzentrierter wässriger Kochsalzlösung gewaschen. Die organische Phase wird dann mit Na₂SO₄ getrocknet, filtriert und das Filtrat zur Trockne eingedampft. Nach einem einstündigen Trocknen des Rückstandes im Hochvakuum bei Raumtemperatur erhält man 485 mg (98 %) der Titelverbindung als farbloses Öl, das noch eine geringe Menge Essigsäureethylester enthält. ¹H-NMR (300 MHz, CDCl₃): 1,04 und 1,05 (2s, 2 CH₃CSi); 1,38 (ddd, J=14,5, 5,0 und 5,0) und 1,47 (ddd, J=3,5, 3,5 und 13,5) und 2,30 (ddd, J=3,0, 8,5 und 14,5) und 2,42 (d, J=13,5), [H₂-C(2) und H₂C(5)]; 1,59 [s, HOCH₂); 3,36 [dd, J=8,5 und 10,5, HOH₂C(6)].

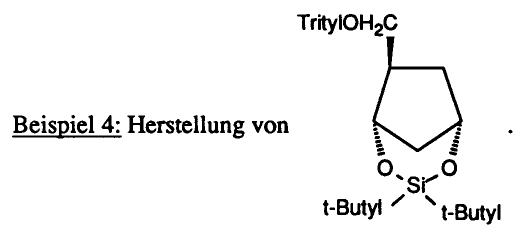

397 mg (1,46 mMol) der Verbindung gemäss Beispiel 3, 17, 8 mg (0,15 mMol) 2-Dimethylaminopyridin, und 405 µl (2,91 mMol) Triethylamin werden unter Argonatmosphäre in 5 ml CH₂Cl₂ gelöst. Dann werden unter Rühren der Lösung bei Raumtemperatur 528 mg (1,89 mMol) Tritylchlorid aufeinmal zugegeben und 18 Stunden weitergerührt. Danach wird das Lösungsmittel im Vakuum abgedampft und der Rückstand mit zweimal 50 ml Hexan/Essigsäureethylester extrahiert. Die Lösung wird darauf mit je 30 ml wässriger 6-prozentiger NaHCO₃ und konzentrierter Kochsalzlösung gewaschen, mit Na₂SO₄ getrocknet, abfiltriert, das Lösungsmittel im Vakuum abgedampft und der Rückstand im Hochvakuum bei Raumtemperatur über Nacht getrocknet. Man erhält 657 mg (88 %) der Titelverbindung. ¹H-NMR (250 MHz, CDCl₃): 1,04 und 1,05 (2s,2 (CH₃CSi); 1,32-1,50 (m) und 2,22 (ddd, J=14,5, 3,0 und 9,0) und 2,35 (d,J=13,5), [H₂-C(2) und H₂C(5)]; 2,66 [m, HC(4)], 4,43-4,58 [m, H-C(1,3)]; 7,17-7,47 [m, 15H-C(aromatisch)].

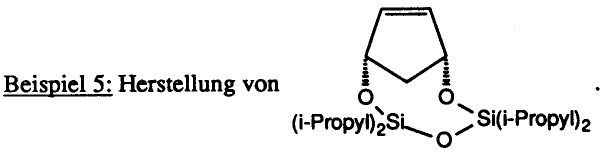

4,05 g (40,5 mmol) cis-4-Cyclopenten-1,3-diol werden unter Argon in 60 ml absolutem Pyridin gelöst und mittels Spritze innerhalb von 15 Minuten bei Raumtemperatur (RT) 14,18 g (45,0 mmol) 1,3-Dichlor-1,1,3,3-tetraisopropyl-disiloxan zugegeben, wobei die Temperatur langsam auf 35°C ansteigt. Die Reaktionsmischung (Pyridiniumhydrochlorid beginnt langsam auszukristallisieren) wird 16 h bei RT gerührt und anschliessend am Rotationsverdampfer eingeengt. Zweimaliges Extrahieren mit Petroleumbenzin aus halbgesättigter NaHCO₃-Lösung, Trocknen der organischen Phase über Na₂SO₄, Filtrieren und Einengen ergeben 10,22 g Rohprodukt, welches im Kugelrohr (130°C, 13 Pa) destillativ gereinigt wird: 8,16 g (75,4%) farbloses Oel. ¹H-NMR (250 MHz, CDCl₃): 6,08 (*s*, H-C(4,5)); 4,82 (d, J = 8,0, H-C(1,3)); 2,54 (dt, J = 15,5, 7,5, H-C(2)); 1,99 (d, J = 15,5, H-C(2)); 0,8-1,1 (m, 4 (CH₃)₂CHSi).

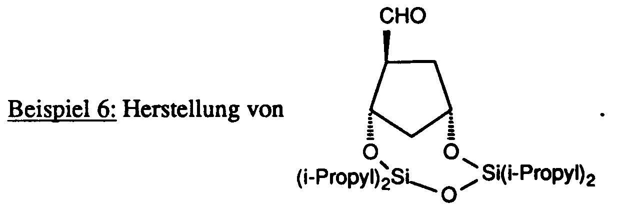

Man verfährt analog zu Beispiel 2. Aus 2,00 g der Verbindung gemäss Beispiel 5 werden nach chromatographischer Reinigung an SiO₂ (Hexan/Diethylether 15:1) 1,93 g (89%) reines Produkt als farbloses, hochviskoses Oel erhalten. ¹H-NMR (250 MHz, CDCl₃): 9,87 (scheinbares s, CHO); 4,84 (dd, J = 7,0, 3,5, H-C(3)); 4,64 (scheinbares t, J = 4,5).

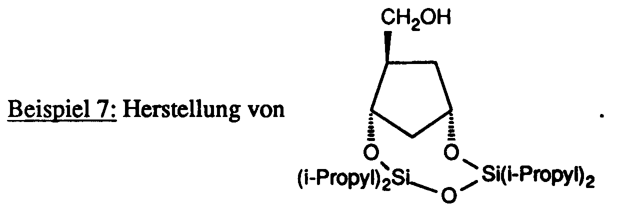

Es wird analog Beispiel 3 verfahren unter Verwendung von 1,69 g der Verbindung gemäss Beispiel 6. Nach der Extraktion werden 1,70 g (100%) Produkt als farbloses, hochviskoses Oel erhalten. ¹H-NMR (250 MHz, CDCl₃): u.a. 4,57 (scheinbares t, J = 4,5) und 4,39 (dd, J = 8,0, 6,0)(H-C(1,3)); 3,73 (J = 6,0, 10,5) und 3,64 (J = 6,5, 10,5)(ABM-System, CH₂OH).

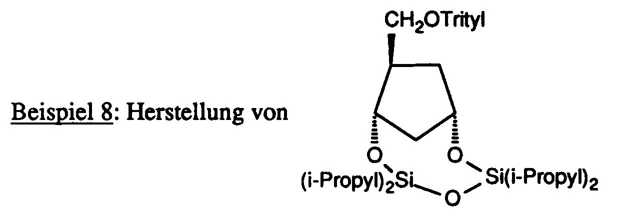

Es wird analog Beispiel 4 verfahren unter Verwendung von 1,60 g Verbindung gemäss Beispiel 7. Nach einer säulenchromatographischen Reinigung an Kieselgel (Petroleumbenzin/Ethylacetat 30:1) erhält man 2,75 g (quantitative Ausbeute) der Titelverbindung als leicht gelbliches Harz. ¹H-NMR (250 MHz, CDCl₃): u.a. 4,52 (scheinbares t, J = 4,0) und 4,30 (dd, J = 7,5, 4,0)(H-C(1,3)); 3,15 (J = 6,5, 9,0) und 3,03 (J = 7,0, 9,0)(ABM-System, CH₂OTr).

### B) Anwendungsbeispiele

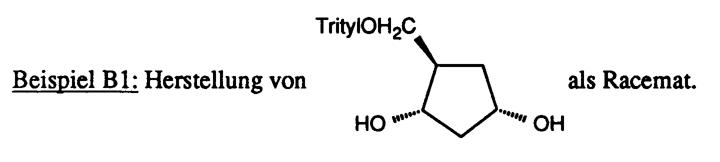

Eine Lösung von 752 mg (2,39 mMol) Tetra-n-butylammoniumfluorid-trihydrat in 10 ml Tetrahydrofuran wird zu 558 mg (1,08 mMol) der Verbindung gemäss Beispiel 4 gegeben und 5 Stunden bei Raumtemperatur gerührt. Man dampft danach das Lösungsmittel im Vakuum ab, löst den Rückstand in Essigsäureethylester/Hexan (2:1) und chromatographiert die Lösung an Silikagel. Von den gesammelten Fraktionen wird das Lösungsmittel im Vakuum abgedampft und dann der Rückstand im Hochvakuum bei Raumtemperatur während 18 Stunden getrocknet. Man erhält 409 mg (quantitative Ausbeute) der Titelverbindung als farblose und harzige Substanz, die beim Stehenlassen kristallisiert. Die Umkristallisation aus Essigsäureethylester/Hexan (1:2) ergibt eine kristalline Substanz mit einem Schmelzpunkt von 116-117 °C. ¹H-NMR (250 MHz, CDCl₃): 1,48 (ddd, J=5,5, 8,8 und 14,0) und 1,78 (m, 2 Hauptsignale) und 1,88-2,08 (m) [H₂-C(2) und H₂-C(5)]; 2,51 [H-C(4)]; 2,03 (d,J=5,0) und 2,58 [d, J=5,0, HO-C(1,3)]; 2,93 [dd, J=8,5 und 8,5, H-C(6)]; 3,21 [dd, J=5,5 und 9,0, H-C(6)]; 4,09 und 4,32 [2m, H-C(1,3)]; 7,18-7,46 [m, H-C(aromatisch)].

Mit 2,75 g der Verbindung gemäss Beispiel 8 verfährt man analog unter Verwendung von 5 Moläquivalenten Tetra-n-butylammoniumfluorid. Nach der säulenchromatographischen Reinigung erhält man 1,56 g (93%) eines zähflüssigen Oels, aus dem man durch Kristallisation 1,23 g (74%) farblose kristalline Substanz gewinnt, die mit der zuvor hergestellten Substanz identisch ist. 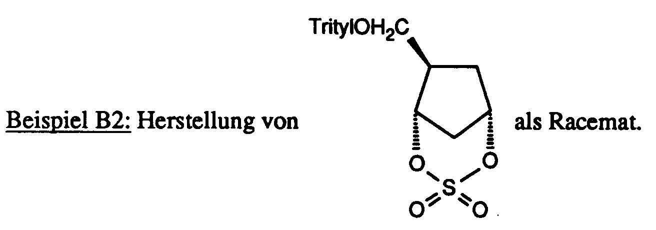
a) 1,049 g (2,80 mMol) der Verbindung gemäss Biespiel B1 werden unter Argonatmosphäre in 20 ml CH₂Cl₂ und 1,56 ml (11,4 mMol) Triethylamin gelöst. Die Lösung wird unter Rühren auf 0 °C gekühlt und 302 µl (4,2 mMol) SOCl₂ während 5 Minuten zugetropft. Die Reaktionsmischung ändert ihre Farbe von farblos über gelb in braun und nach 10 Minuten fällt ein brauner Niederschlag aus. Darauf wird das Reaktionsgemisch zweimal mit 20 ml eiskaltem Wasser und dann mit 30 ml eiskalter Kochsalzlösung (18 Volumen-%) extrahiert. Die organische Phase wird dann über MgSO₄ getrocknet, filtriert, und bei 15 °C im Vakuum zur Trockne eingedampft. Der Rückstand wird zweimal in je 6 ml CH₃CN gelöst, wieder zur Trockne eingedampft und im Hochvakuum 10 Minuten getrocknet. Man erhält 1,335 g der Titelverbindung (113 %, Gehalt an CH₃CN und Triethylamin als Verunreinigungen), die so im nachfolgenden Verfahren b) verwendet werden.
b) 8,50 g der nach a) hergestellten Verbindung werden in 160 ml CH₃CN/CCl₄ (1:1) gelöst und auf 0 °C gekühlt. Dann fügt man nacheinander 120 ml Wasser, 47 mg (200 µmol) RuCl₃xH₂O und 5,71 g (26,7 mMol) NaIO₄ zu. Die Mischung wird während einer Stunde kräftig gerührt, danach 300 ml Diethylether zugefügt und die wässrige Phase abgetrennt. Die organische Phase wird dreimal mit 100 ml eiskalter und konzentrierter wässriger Kochsalzlösung gewaschen, über MgSO₄ getrocknet, abfiltriert, im Vakuum bei 15 °C eingedampft und dann im Hochvakuum getrocknet. Man erhält 5,80 g (99,5 %, bezogen auf die beiden Verfahrensstufen) der Titelverbindung als farblosen und amorphen Feststoff. ¹H-NMR (250 MHz, CDCl₃): 1,61 (dt, J=13,5 und 4,0) und 1,77 (dt, J=12,5 und 2,5) und 2,69 (d, J=12,5) und 2,80-2,92 (m, teilweise überlappende Signale) [H₂-C(2) und H₂-C(5)]; 3,39 [m, H-C(4)]; 2,84 (dd, J=8,5 und 7,5,) und 3,29 [dd, J=8,5 und 4,5, H₂-C(6)]; 5,09 (breites s) und 5,22 [breites s, H-C(1,3)]; 7,21-7,45 [m, H-C(aromatisch)].

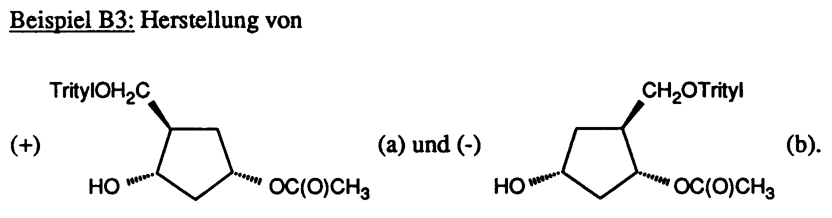

6,22 g (16,6 mMol) der Verbindung gemäss Beispiel B1 werden in 63 ml Vinylacetat gelöst und mit 1,24 g Pseudomonas fluorescens Lipase (PFL) versetzt. Die heterogene Reaktionsmischung wird darauf 50 Stunden bei Raumtemperatur gerührt. Danach wird die Mischung im Vakuum eingedampft, in Hexan/Essigsäureethylester (2:1) aufgenommen und dann an Silikagel chromatographiert. Man erhält 250 mg der Diacetatverbindung, 3,38 g reine Verbindung (a) und 3,45 g reine Verbindung (b) sowie 970 mg nicht getrennter Monoacetate, die nochmals chromatographiert werden. Die vereinigten reinen Verbindungen (a) und (b) werden dreimal in CH₃CN gelöst und im Vakuum zur Trockene eingedampft. Man erhält 3,00 g (43,4 %) der Verbindung (a) und 3,15 g (45,5 %) der Verbindung (b) als leicht gelbe Öle, die noch mit etwas CH₃CN verunreinigt sind.
¹H-NMR (250 MHz, CDCl₃) der Verbindung (a), (1S,3S,4R)-Trans-4-trityloxy-cis-3-hydroxy-1-acetoxy-cyclopentan: 1,54 [m, H-C(5)]; 1,74 [m, H₂C(2)]; 1,89 [m, H-C(5)]; 2,05 (s,H₃CCOO); 2,40 [m, H-C(2) und H-C(4)]; 2,52 [d, J=4,0, HO-C(3)]; 2,98 [t, J=8,5, H-C(6)]; 3,34 [dd, J=9,0 und 5,0, H-C(6)]; 3,95 [dq, J=3,0 und 7,0, H-C(3)]; 5,07 [m, H(1)]; 7,21-7,45 [m, H-C(aromatisch)]. Die Verbindung (a) wird mit einer optischen Reinheit von ee gleich 98,2 % erhalten (bestimmt mit HPLC, Chiracel® OD, Hexan/i-Propanol 9:1, Fliessrate 1 ml/Minute).
¹H-NMR (250 MHz, CDCl₃) der Verbindung (a), (1R,3R,4S)-Trans-4-trityloxy-cis-1-hydroxy-3-acetoxy-cyclopentan: 1,61-178 [m, H-C(2,5) und HO-C(1)]; 1,95 [m, H-C(5)]; 2,03 [s, H₃-CCOO]; 2,30 [ddd, J=14,5 und 7,5 und 5,5, H-C(2)]; 2,60 [m, H-C(4)]; 3,12 [ABM-System, H₂-C(6)]; 4,34 [m, H-C(1)]; 5,05 [m, H-C(3)]; 7,18-7,45 [m, H-C(aromatisch)]. Die Verbindung (b) wird mit einer optischen Reinheit von ee 98,6 % erhalten.

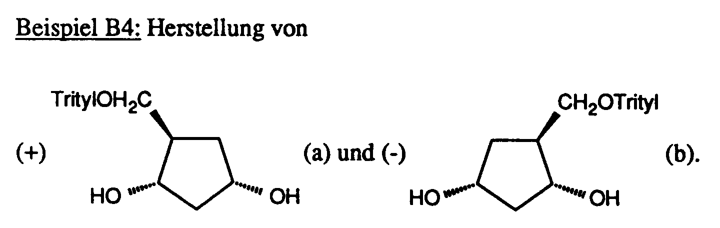

100 g (240 mmol) der Monoacetate gemäss Beispiel B3 werden unter Argon in 600 ml Methanol gelöst und 188,5 g (4,8 mol) Ethylendiamin zugegeben, wobei die Temperatur auf 50 °C ansteigt. Das Reaktionsgemisch wird 15 h bei dieser Temperatur gerührt. Danach wird das Lösungsmittel im Hochvakuum abgedampft und der Rückstand dreimal in Acetonitril aufgenommen und wieder eingedampft. Das erhaltene Öl wird in 500 ml Ethylacetat gelöst, 300 ml Wasser zugegeben und unter Rühren wässrige Zitronensäurelösung (10 %) zugefügt. Nach Zugabe von weiteren 500 ml Ethylacetat wird die organische Phase abgetrennt und die wässrige Phase zweimal mit je 150 ml Ethylacetat extrahiert. Die vereinigten organischen Phasen werden über NaHCO₃ getrocknet und dann eingedampft. Der Rückstand wird zweimal in Acetonitril aufgenommen und eingedampft und dann aus Cyclohexan (1 Woche, 8-10 °C) umkristallisiert. Nach dem Trocknen erhält man je 85,2 g (95 %) der Titelverbindungen als farblose Kristalle. Die ¹H-NMR-Spektren sind mit dem Racemat gemäss Beispiel B1 identisch.
Verbindung (a): Schmelzpunkt 105-106 °C; [α]²⁵= +24,1 (589, c = 1,0, CH₃OH), +73,7 (365 nm).
Verbindung (b): Schmelzpunkt 105-106 °C; [α]²⁵= -25,8 (589, c = 1,0, CH₃OH), - 75,2 (365 nm).

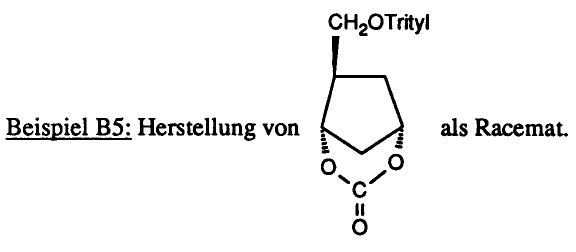

200 mg (534 µmol) der Verbindung gemäss Beispiel B1 werden in Toluol aufgeschlämmt, wenig Molekularsieb (3Å) zugegeben und etwa 5 min unter Argon gerührt. 5 min nach der Zugabe von 95,3 mg (588 µmol) Carbonyldiimidazol wird die Reaktionsmischung klar. Nach 36 h Rühren bei 90°C und 45 Tagen bei Raumtemperatur wird am Rotationsverdampfer eingeengt und das Reaktionsgemisch an Kieselgel gereinigt (Essigsäureethylester/Hexan 1:2). Die reinen Produktfraktionen werden vereinigt und eingeengt, dann in CH₃CN aufgenommen und erneut eingeengt. Man erhält 53 mg (25%) reines Produkt. ¹H-NMR (250 MHz, CDCl₃): u.a. 4,84 (m) und 4,78 (m)(H-C(1,3)); 3,22 (quartettartiges m, H-C(4)); 2,84 (m, CH₂OTr). 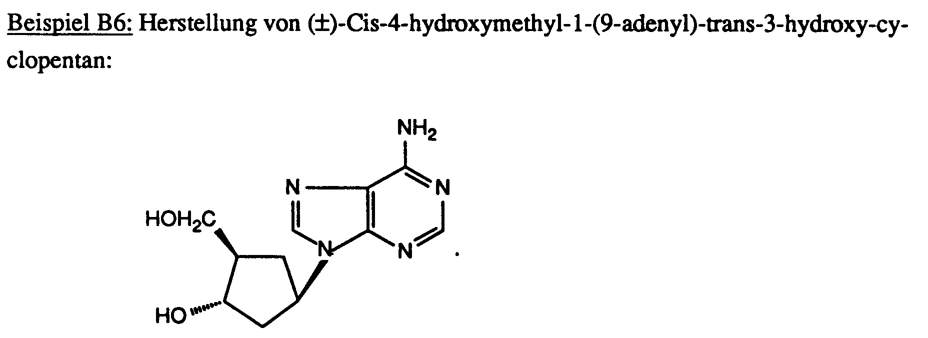
a) 297 mg (2,20 mMol) Adenin werden unter Argonatmosphäre in 10 ml CH₃CN suspendiert und dann 337 mg (2,21 mMol) 1,8-Diaza-bicyclo-[5,4,0]-undec-7-en zugegeben. Man rührt die Mischung 15 Minuten bei Raumtemperatur und versetzt anschliessend mit 0,50 g (1,15 mMol) des Racemats gemäss Beispiel B2. Danach wird die Reaktionsmischung noch 15 Stunden bei Raumtemperatur gerührt. Nach dem Abdampfen des Lösungsmittels im Vakuum wird der Rückstand in CH₂Cl₂/Methanol/Triethylamin (30:3:1) aufgenommen und an Silikagel chromatographiert. Die vereinten Fraktionen werden zur Trockne eingedampft und der Rückstand zweimal in je 20 ml CH₃CN gelöst und wieder zur Trockne eingedampft. Der Rückstand wird dann in 30 ml Wasser gelöst und mit Triethylamin versetzt. Der Niederschlag wird abfiltriert und getrocknet. Man erhält 590 mg (76 %) des Tetraethylammoniumsalzes von (±)-Cis-4-Trityloxymethyl-1-(9-adenyl)-trans-3-sulfato-cyclopentan als farbloses Pulver. ¹H-NMR (250 MHz, CD₃OD): 1,31 [t, J=7,0, (CH₃CH₂)₃NH⁺]; 3,20 [q, J=7,0, (CH₃CH₂)₃NH⁺]; 1,64-2,66 [m, H₂-C(2',5') und H-C(4')]; 3,15-3,40 [m, H₂-C(6')]; 5,02-5,17 [m,H-C(1',3')]; 7,18-7,51 [m, H-C(Trityl)]; 8,15 und 8,16 [2s, H-C(Adenin)].
b) 200 mg der gemäss a) hergestellten Verbindung werden in je 10 ml 2N HCl und Ethanol gelöst und 24 Stunden bei 80-85 °C erhitzt. Danach wird das Lösungsmittel im Vakuum abgedampft und der Rückstand zweimal in je 20 ml CH₃CN aufgenommen und eingedampft. Das erhaltene Rohprodukt wird in Methanol/Essigsäureethylester (1:1) aufgenommen und an Silikagel chromatographiert. Nach dem Abdampfen des Lösungsmittels wird der Rückstand mit dem gleichen Lösungsmittel umkristallisiert. Man erhält 80,1 mg (94 %) (±)-Cis-4-Hydroxymethyl-1-(9-adenyl)-trans-3-hydroxy-cyclopentan-Hydrochlorid als farblose Kristalle. ¹³C-NMR (63 MHz, CD₃OD): 35,1 (C(2')), 41,7 (C(6')), 50,7 (C(4')), 55,9 (C(1')), 64,2 (C(5')), 73,7 (C(3')), 120,3, 150,3 and 151,7 (C(4',5',6')), 144,3 and 144,7 (HC(2,8)).
c) 400 mg (1,40 mmol) der gemäss b) erhaltenen Verbindung wird in Methanol gelöst und über eine Kolonne mit 10 g Amberlit IRA-93 (OH-Form) filtriert. Die gesammelten Fraktionen werden im Vakuum eingedampft und zweimal mit CH₃CN aufgenommen und eingedampft. Der Rückstand wird in Methanol gelöst, zum Rückfluss erhitzt und Essigsäuremethylester zugegeben, bis die Mischung leicht trüb wird. Dann lässt man über Nacht bei 4 °C kristallisieren, filtriert die Kristalle ab und trocknet im Hochvakuum. Man erhält 320 mg (92%) der Titelverbindung als farblose Kristalle mit einen Schmelzpunkt von 183 bis 185 °C. ¹H-NMR (250 MHz, CD₃OD): 1,94 (dt, J = 12,5, 10,0) and 2,14-2,66 (m)(H₂-C(2',6') and H-C(4')); 3,72 (ABM-system, H₂-C(5')); 4,32 (m, H-C(3')); 5,24 (quint.-like m, H-C(1')); 8,41 (s) and 8,48 (s)(H-C(2,8)).

### Beispiel B7: Herstellung von (1R,3S,4R)-Cis-4-hydroxymethyl-trans-3-hydroxy-1-(9'-adenyl)-cyclopentan-Hydrochlorid.

Die Verbindung (a) von Beispiel B4 wird gemäss Beispiel B2 in das cyclische Sulfat übergeführt und 11,50 g (26,3 mmol) gemäss Beispiel B6 mit 3,76 g (27,8 mmol) Adenin umgesetzt. Man erhält 14,25 g (80,4 %) der kristallinen Titelverbindung. ¹H-NMR (250 MHz, CD₃OD): 1,94 (dt, J = 12,5, 10,0) and 2,14-2,66 (m)(H₂-C(2',6') and H-C(4')); 3,72 (ABM-system, H₂-C(5')); 4,32 (m, H-C(3')); 5,24 (quintettartiges m, H-C(1')); 8.41 (s) und 8,48 (s)(H-C(2,8)).

### Beispiel B8: Herstellung von (1S,3R,4S)-Cis-4-hydroxymethyl-trans-3-hydroxy-1-(9'-adenyl)-cyclopentan.

Es wird gemäss Beispiel B7 verfahren, aber mit Verbindung (b) von Beispiel B4. Man erhält 76,9 % der Titelverbindung als farblose Kristalle, Schmelzpunkt 185-186 °C, [α]²⁵= - 10,2 (589, c = 0,5, H₂O).

## Patentansprüche

1. Verbindungen der Formeln Ia und Ib 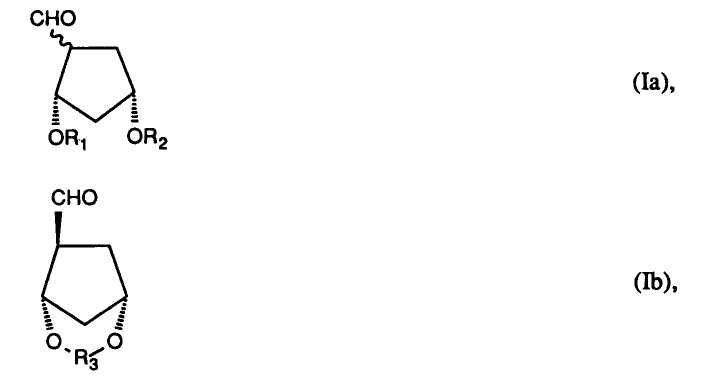 worin R₁ und R₂ unabhängig voneinander lineares oder verzweigtes C₁-C₁₂-Alkyl, Cycloalkyl mit 5 bis 8 Ringkohlenstoffatomen, Phenylalkyl mit 1 bis 4 C-Atomen in der Alkylengruppe bedeuten, wobei die cyclischen Reste unsubstituiert oder mit C₁-C₄-Alkyl oder C₁-C₄-Alkoxy substituiert sind, oder R₁ und R₂ unabhängig voneinander -SiR₄R₅R₆ darstellen, worin R₄, R₅ und R₆ unabhängig voneinander C₁-C₁₂-Alkyl, Phenyl, Benzyl oder mit C₁-C₄-Alkyl oder C₁-C₄-Alkoxy substituiertes Phenyl oder Benzyl darstellen, und R₃ eine Brückengruppe bedeutet, die zusammen mit dem Rest -OCH-CH₂-CHO-, an den sie gebunden ist, einen 6- bis 8-gliedrigen Ring bildet.

2. Verbindungen gemäss Anspruch 1, dadurch gekennzeichnet, dass in Formel Ia R₁ und R₂ gleiche Reste darstellen.

3. Verbindungen gemäss Anspruch 2, dadurch gekennzeichnet, dass in Formel Ia R₁ und R₂ gleiche Reste -SiR₄R₅R₆ darstellen, worin R₄, R₅ und R₆ die in Anspruch 1 angegebenen Bedeutungen haben.

4. Verbindungen gemäss Anspruch 3, dadurch gekennzeichnet, dass R₄, R₅ und R₆ C₁-C₁₂-Alkyl oder Phenyl bedeuten.

5. Verbindungen gemäss Anspruch 1, dadurch gekennzeichnet, dass R₃ in Formel Ib für Alkyliden mit 1 bis 20 C-Atomen, Cycloalkyliden mit 5 bis 8 Ringkohlenstoffatomen, oder Reste der Formeln R₇R₈Si= und -SiR₇R₈-O-R₇R₈Si- steht, worin R₇ und R₈ unabhängig voneinander C₁-C₁₂-Alkyl, Phenyl, Benzyl oder mit C₁-C₄-Alkyl oder C₁-C₄-Alkoxy substituiertes Phenyl oder Benzyl darstellen.

6. Verbindungen gemäss Anspruch 5, dadurch gekennzeichnet, dass R₃ als Alkyliden 1 bis 14 C-Atome enthält.

7. Verbindungen gemäss Anspruch 5, dadurch gekennzeichnet, dass R₇ und R₈ unabhängig voneinander C₁-C₁₂-Alkyl oder Phenyl bedeuten.

8. Verbindungen gemäss Anspruch 1, dadurch gekennzeichnet, dass es sich um die Verbindungen der Ib handelt.

9. Verbindungen gemäss Anspruch 8, dadurch gekennzeichnet, dass R₃ für Reste der Formeln R₇R₈Si= oder -SiR₇R₈-O-R₇R₈Si- steht, worin R₇ und R₈ unabhängig voneinander die in Anspruch 1 angegebenen Bedeutungen haben.

10. Verbindungen gemäss Anspruch 9, dadurch gekennzeichnet, dass R₇ und R₈ gleiche Reste darstellen und für C₁-C₆-Alkyl stehen.

11. Verfahren zur Herstellung der Verbindungen der Formeln Ia und Ib, dadurch gekennzeichnet, dass man
a) in einer ersten Stufe 1 Äquivalent Cis-1,3-Pent-4-endiol der Formel 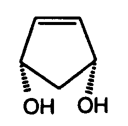
a1) mit 2 Äquivalenten R₁X, R₂X oder Mischungen hiervon umsetzt, oder
a2) mit einem Äquivalent XR₃X umsetzt, worin R₁, R₂ und R₃ die zuvor angegebene Bedeutung haben und X oder zwei X gemeinsam für eine Abgangsgruppe steht, und
b) in einer zweiten Stufe die erhaltenen Verbindungen der Formeln IIa und IIb 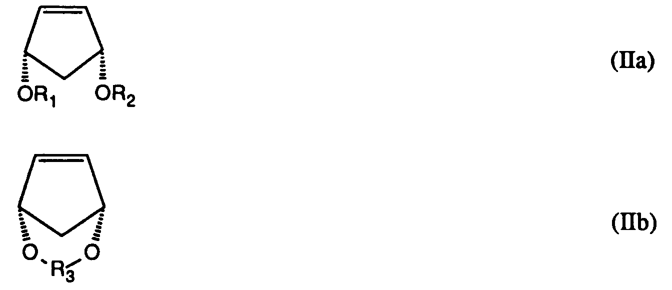 in Gegenwart eines Katalysators bei erhöhter Temperatur und unter Druck mit einem Gemisch aus H₂ und CO zu Verbindungen der Formeln Ia und Ib hydroformyliert.

12. Verfahren gemäss Anspruch 11, dadurch gekennzeichnet dass es sich bei der Abgangsgruppe X um Halogenid, sowie CF₃COO^{⊖}, RSO₃^{⊖}, SO₄^{2⊖}, PO₄^{3⊖}, CH₃CO₂^{⊖}, OH^{⊖} handelt, worin R für C₁-C₆-Alkyl oder teilweise oder vollständig mit Fluor substituiertes C₁-C₆-Alkyl, oder unsubstituiertes oder mit C₁-C₄-Alkyl, C₁-C₄-Alkoxy oder Halogen substituiertes Phenyl oder Benzyl bedeutet.

13. Verfahren gemäss Anspruch 11, dadurch gekennzeichnet dass es sich bei den Verbindungen, worin zwei X eine Abgangsgruppe darstellen, um Aldehyde oder Ketone handelt.

14. Verfahren gemäss Anspruch 11, dadurch gekennzeichnet dass es sich bei den Katalysatoren in der Verfahrensstufe b) um komplexierte Edelmetallsalze handelt.

15. Verbindungen der Formel IIb 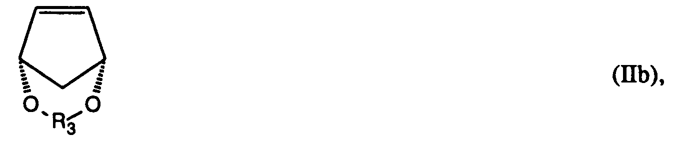 worin R₃ die in Anspruch 1 angegebene Bedeutung hat.

16. Verbindungen gemäss Anspruch 15, dadurch gekennzeichnet, dass R₃ für Reste der Formeln R₇R₈Si= oder -SiR₇R₈-O-R₇R₈Si- steht, worin R₇ und R₈ unabhängig voneinander die in Anspruch 1 angegebenen Bedeutungen haben.

17. Verbindungen der Formeln IIIa und IIIb 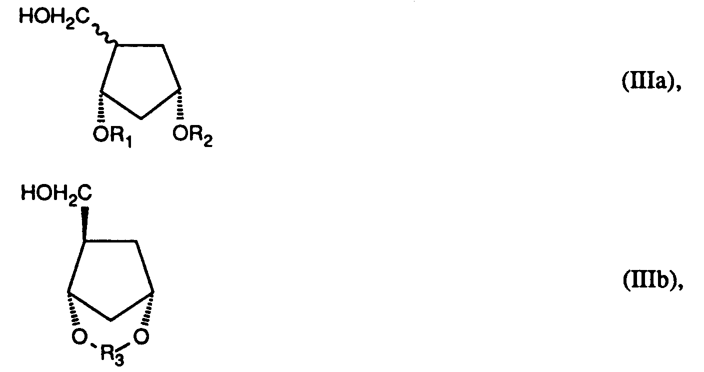 worin R₁, R₂ und R₃ die in Anspruch 1 angegebenen Bedeutungen haben, in Form Ihrer Racemate und Enantiomeren, sowie deren hydroxylgeschützte Derivate.

18. Verbindungen gemäss Anspruch 17, dadurch gekennzeichnet, dass die geschützten Derivate eine abspaltbare Schutzgruppe aus der Gruppe lineares oder verzweigtes C₁-C₈-Alkyl, C₇-C₁₂-Aralkyl, Diphenylmethyl, Trityl, Trialkylsilyl mit 3 bis 20 C-Atomen, C₂-C₁₂-Acyl oder R₁₀-SO₂- enthalten, worin R₃ C₁-C₁₂-Alkyl, C₅- oder C₆-Cycloalkyl, Phenyl, Benzyl, C₁-C₁₂-Alkylphenyl, oder C₁-C₁₂-Alkylbenzyl, oder Halogenphenyl oder Halogenbenzyl oder C₁-C₁₂-Alkoxycarbonyl bedeutet.

19. Verbindungen gemäss Anspruch 17, dadurch gekennzeichnet, dass es sich um die Verbindungen der Formel IIIb handelt.

20. Verbindungen gemäss Anspruch 19, dadurch gekennzeichnet, dass R₃ für Reste der Formeln R₇R₈Si= oder -SiR₇R₈-O-R₇R₈Si- steht, worin R₇ und R₈ unabhängig voneinander die in Anspruch 1 angegebenen Bedeutungen haben.

## Claims

1. A compound of the formula Ia or Ib 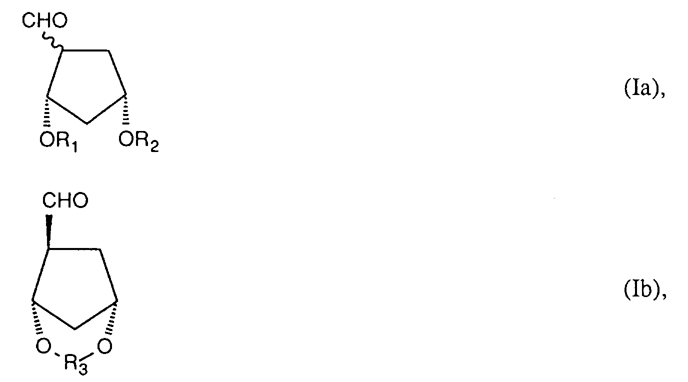 in which R₁ and R₂ independently of one another are linear or branched C₁-C₁₂alkyl, cycloalkyl having 5 to 8 ring carbon atoms, phenylalkyl having 1 to 4 C atoms in the alkylene group, the cyclic radicals being unsubstituted or substituted by C₁-C₄alkyl or C₁-C₄alkoxy, or R₁ and R₂ independently of one another are -SiR₄R₅R₆ in which R₄, R₅ and R₆ independently of one another are C₁-C₁₂alkyl, phenyl, benzyl, or phenyl or benzyl, each of which is substituted by C₁-C₄alkyl or C₁-C₄alkoxy, and R₃ is a bridging member which, together with the radical -OCH-CH₂-CHO- to which it is bonded, forms a 6- to 8-membered ring.

2. A compound according to claim 1, wherein, in formula Ia, R₁ and R₂ are identical radicals.

3. A compound according to claim 2, wherein, in formula Ia, R₁ and R₂ are identical radicals -SiR₄R₅R₆ in which R₄, R₅ and R₆ are as defined in claim 1.

4. A compound according to claim 3, wherein R₄, R₅ and R₆ are C₁-C₁₂alkyl or phenyl.

5. A compound according to claim 1, wherein R₃ in formula Ib is alkylidene having 1 to 20 C atoms, cycloalkylidene having 5 to 8 ring carbon atoms, or radicals of the formulae R₇R₈Si= and -SiR₇R₈-O-R₇R₈Si- in which R₇ and R₈ independently of one another are C₁-C₁₂alkyl, phenyl, benzyl or phenyl or benzyl which are substituted by C₁-C₄alkyl or C₁-C₄alkoxy.

6. A compound according to claim 5, wherein R₃ as alkylidene contains 1 to 14 C atoms.

7. A compound according to claim 5, wherein R₇ and R₈ independently of one another are C₁-C₁₂alkyl or phenyl.

8. A compound according to claim 1, which is a compound of the formula Ib.

9. A compound according to claim 8, wherein R₃ is a radical of the formula R₇R₈Si= or -SiR₇R₈-O-R₇R₈Si- in which R₇ and R₈ independently of one another are as defined in claim 1.

10. A compound according to claim 9, wherein R₇ and R₈ are identical radicals and are C₁-C₆alkyl.

11. A process for the preparation of a compound of the formula Ia or Ib, which comprises
a) reacting, in a first step, 1 equivalent of cis-1,3-pent-4-enediol, of the formula 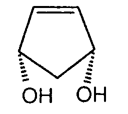
a1) with 2 equivalents of R₁X, R₂X or mixtures of these, or
a2) with one equivalent of XR₃X, in which R₁, R₂ and R₃ are as defined above and X or two X together are a leaving group, and
b) hydroformylating the resulting compounds of the formulae IIa and IIb 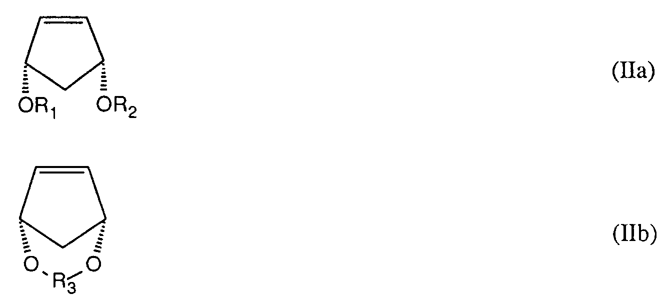 in a second step in the presence of a catalyst at increased temperature and under pressure with a mixture of H₂ and CO to give compounds of the formulae Ia and Ib.

12. A process according to claim 11, wherein the leaving group X is a halide, as well as CF₃COO^{⊖}, RSO₃^{⊖}, SO₄^{2⊖}, PO₄^{3⊖}, CH₃CO₂^{⊖}, OH^{⊖}, in which R is C₁-C₆alkyl or partially or completely fluorine-substituted C₁-C₆alkyl, or is phenyl or benzyl which are unsubstituted or substituted by C₁-C₄alkyl, C₁-C₄alkoxy or halogen.

13. A process according to claim 11, wherein the compounds in which two X are a leaving group are aldehydes or ketones.

14. A process according to claim 11, wherein the catalysts in process step b) are complexed noble metal salts.

15. A compound of the formula IIb 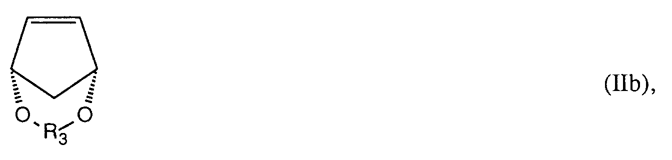 in which R₃ is as defined in claim 1.

16. A compound according to claim 15, wherein R₃ is a radical of the formulae R₇R₈Si= or -SiR₇R₈-O-R₇R₈Si- in which R₇ and R₈ independently of one another are as defined in claim 1.

17. A compound of the formula IIIa or IIIb 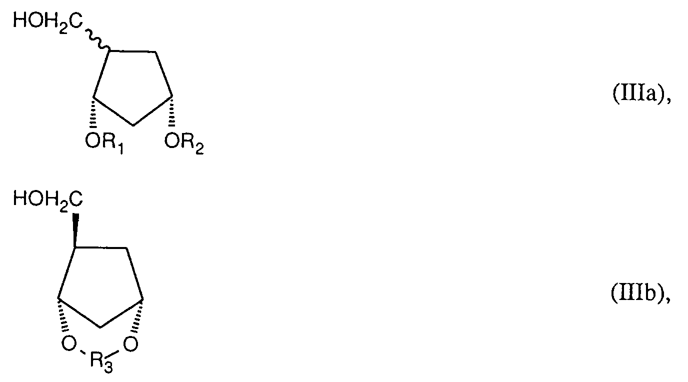 in which R₁, R₂ and R₃ are as defined in claim 1, in the form of a racemate thereof or enantiomer thereof, as well as a hydroxyl-protected derivative thereof.

18. A compound according to claim 17, wherein the protected derivative comprises a detachable protective group from the group comprising linear or branched C₁-C₈alkyl, C₇-C₁₂aralkyl, diphenylmethyl, trityl, trialkylsilyl having 3 to 20 C atoms, C₂-C₁₂acyl or R₁₀-SO₂-, in which R₃ is C₁-C₁₂alkyl, C₅- or C₆cycloalkyl, phenyl, benzyl, C₁-C₁₂alkylphenyl or C₁-C₁₂alkylbenzyl, or halophenyl or halobenzyl or C₁-C₁₂alkoxycarbonyl.

19. A compound according to claim 17, which is a compound of the formula IIIb.

20. A compound according to claim 19, wherein R₃ is a radical of the formula R₇R₈Si= or -SiR₇R₈-O-R₇R₈Si-, in which R₇ and R₈ independently of one another are as defined in claim 1.

## Revendications

1. Composés répondant aux formules Ia et Ib 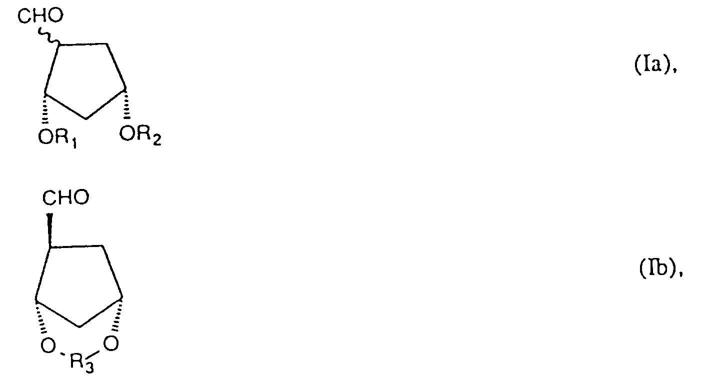 dans lesquelles R₁ et R₂ représentent chacun, indépendamment l'un de l'autre, un groupe alkyle à chaîne droite ou ramifiée en C1-C12, un groupe cycloalkyle en C5-C8, un groupe phénylalkyle contenant un à quatre atomes de carbone dans la partie alkyle, les groupes cycliques étant non substitués ou substitués par des groupes alkyle en C1-C4 ou alcoxy en C1-C4, ou bien R₁ et R₂ représentent chacun, indépendamment l'un de l'autre, un groupe -SiR₄R₅R₆ dans lequel R₄, R₅ et R₆ représentent chacun, indépendamment les uns des autres, un groupe alkyle en C1-C12, phényle, benzyle ou un groupe phényle ou benzyle substitué par des groupes alkyle en C1-C4 ou alcoxy en C1-C4, et R₃ représente un pont qui, avec le groupe -OCH-CH₂-CHO- auquel il est relié, forme un cycle de six à huit chaînons.

2. Composés selon revendication 1, caractérisés en ce que les substituants R₁ et R₂ de la formule Ia sont identiques.

3. Composés selon revendication 2, caractérisés en ce que, dans la formule Ia, R₁ et R₂ représentent des groupes -SiR₄R₅R₆ identiques, R₄, R₅ et R₆ ayant des significations indiquées dans la revendication 1.

4. Composés selon revendication 3, caractérisés en ce que R₄, R₅ et R₆ représentent des groupes alkyle en C1-C12 ou phényle.

5. Composés selon revendication 1, caractérisé en ce que, dans la formule Ib, R₃ représente un groupe alkylidène en C1-C20, cycloalkylidène en C5-C8, ou bien des groupes de formules R₇R₈Si= et -SiR₇R₈-O-R₇R₈Si- dans lesquelles R₇ et R₈ représentent chacun, indépendamment l'un de l'autre, un groupe alkyle en C1-C12, phényle, benzyle ou phényle ou benzyle portant des substituants alcoxy en C1-C4.

6. Composés selon revendication 5, caractérisés en ce que R₃ représente un groupe alkylidène en C1-C14.

7. Composés selon revendication 5, caractérisés en ce que R₇ et R₈ représentent chacun, indépendamment l'un de l'autre, un groupe alkyle en C1-C12 ou phényle.

8. Composés selon revendication 1, caractérisés en ce qu'il s'agit de composés de formule Ib.

9. Composés selon revendication 8, caractérisés en ce que R₃ représente des groupes de formules R₇R₈Si= et -SiR₇R₈-O-R₇R₈Si- dans lesquelles R₇ et R₈ ont chacun, indépendamment l'un de l'autre, les significations indiquées dans la revendication 1.

10. Composés selon revendication 9, caractérisés en ce que R₇ et R₈ sont identiques et représentent des groupes alkyle en C1-C6.

11. Procédé de préparation des composés de formules Ia et Ib, caractérisé en ce que
a) dans un premier stade d'opération, on fait réagir un équivalent du cis-1,3-penta-4-ène-diol de formule 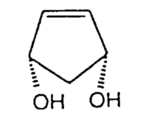
a1) avec deux équivalents de R₁X, R₂X ou leurs mélanges, ou bien
a2) avec un équivalent de XR₃X, R₁, R₂ et R₃ ayant les significations indiquées ci-dessus et X représente un groupe éliminable ou deux X représentent ensemble un substituant éliminable, et
b) dans un deuxième stade opératoire, on soumet les composés obtenus, répondant aux formules IIa et IIb 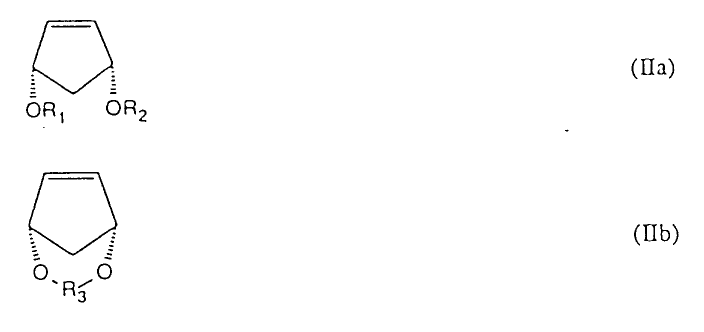 à hydroformylation en présence d'un catalyseur à température élevée et sous pression à l'aide d'un mélange de H₂ et de CO, ce qui donne les composés de formules Ia et Ib.

12. Procédé selon revendication 11, caractérisé en ce que le groupe éliminable X consiste en un halogénure ou en CF₃COO⁻, RSO₃⁻, SO₄²⁻, PO₄³⁻, CH₃CO₂⁻, OH⁻, dans lesquels R représente un groupe alkyle en C1-C6 ou un groupe alkyle en C1-C6 partiellement ou totalement substitué par le fluor, ou un groupe phényle ou benzyle non substitué ou substitué par des groupes alkyle en C1-C4, alcoxy en C1-C4 ou des halogènes.

13. Procédé selon revendication 11, caractérisé en ce que les composés pour lesquels deux X forment ensemble un groupe éliminable sont des aldéhydes ou cétones.

14. Procédé selon revendication 11, caractérisé en ce que les catalyseurs du stade opératoire b) sont des sels complexes de métaux nobles.

15. Composés de formule IIb 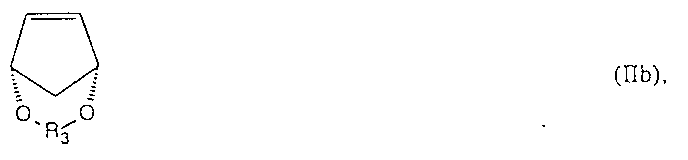 dans laquelle R₃ a les significations indiquées dans la revendication 1.

16. Composés selon revendication 15, caractérisés en ce que R₃ représente des groupes de formules R₇R₈Si= ou -SiR₇R₈-O-R₇R₈Si- dans lesquelles R₇ et R₈ ont chacun, indépendamment l'un de l'autre, les significations indiquées dans la revendication 1.

17. Composés de formules IIIa et IIIb 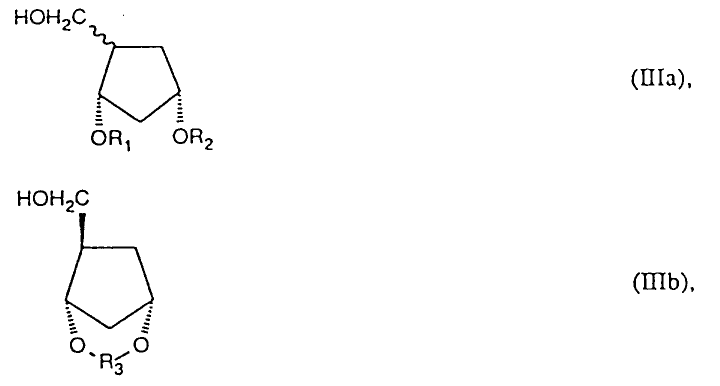 dans lesquelles R₁, R₂ et R₃ ont les significations indiquées dans la revendication 1, à l'état de racémates et d'énantiomères, et leurs dérivés protégés sur les groupes hydroxy.

18. Composés selon revendication 17, caractérisés en ce que les dérivés protégés portent un groupe protecteur éliminable choisi parmi les groupes alkyle à chaîne droite ou ramifiée en C1-C8, les groupes aralkyle en C7-C12, diphénylméthyle, trityle, trialkylsilyle contenant trois à vingt atomes de carbone, acyle en C2-C12 ou R₁₀-SO₂-, R₃ représentant un groupe alkyle en C1-C12, cycloalkyle en C5 ou C6, phényle, benzyle, (alkyle en C1-C12)phényle ou (alkyle en C1-C12)benzyle ou halogénophényle ou halogénobenzyle ou (alcoxy en C1-C12)carbonyle.

19. Composés selon revendication 17, caractérisés en ce qu'il s'agit de composés de formule IIIb.

20. Composés selon revendication 19, caractérisés en ce que R₃ représente des groupes de formules R₇R₈Si= ou -SiR₇R₈-O-R₇R₈Si- dans lesquelles R₇ et R₈ ont chacun, indépendamment l'un de l'autre, les significations indiquées dans la revendication 1.
